# EUROPEAN PATENT SPECIFICATION

(11) **EP 0 909 145 B1**
(45) Date of publication and mention of the grant of the patent: **25.09.2002**
(21) Application number: 97926001.5
(22) Date of filing: 12.06.1997
(51) Int. Cl.: A61B 17/86

(54) **ORTHOPAEDIC FIXATOR PIN ASSEMBLIES**
ORTHOPÄDISCHE FIXATIONSSTIFT-BAUGRUPPEN
ENSEMBLES BROCHES POUR FIXATEURS ORTHOPEDIQUES

(30) Priority: 13.06.1996 GB 9612355; 18.07.1996 GB 9615064
(43) Date of publication of application: 21.04.1999
(73) Proprietor: Karnezis, Ioannis, Bristol BS2 8HV (GB)
(72) Inventor: Karnezis, Ioannis, Bristol BS2 8HV (GB)
(74) Representative: Böhme, Ulrich, Dr. Dipl.-Phys.
(86) International application number: EP9703054
(87) International publication number: WO97047252

(56) References cited:
- EP-A- 0 241 914
- US-A- 4 456 005

## Description

The present invention relates to an orthopaedic device which comprises a plurality of pins adapted to be inserted into a bone of a patient so as to provide anchor points for one or more fixator structures disposed within the patient's body and spaced from the patient's bones.

At present one use of orthopaedic apparatus involves the process of inserting pins to the bones of the patient to provide anchor points to which external fixator systems may be attached (EP 0 241 914 A2). Holes are drilled through the bone and the pins are then screwed into the holes. With the present apparatus the bone may be damaged during the drilling and pin insertion processes due to excessive frictional temperatures. Also the pins have a tendency to slacken off and become loose within the bone structure. The loose pin can cause further damage to the bone structure and lessens the effectiveness of the bracing.

Another known apparatus comprises an internal fixator plate which is secured by screws in face contact with a bone.

According to the present invention an orthopaedic device is provided which comprises one or more fixator structures and a plurality of pins adapted to be inserted into a bone of a patient so as to provide anchor points for one or more fixator structures adapted to be disposed within the patient's body and spaced from the patient's bones, characterised in that the pins employed comprise an outer tubular shaft and an inner shaft extending coaxially therethrough, each shaft comprising a respective externally-threaded securing means for threadedly engaging with a bone, the securing means being axially adjustable relative to each other.

Pins comprising an outer tubular shaft and an inner shaft of this kind are known per se from US 4,456,005. However, these pins are only used to provide compression on a bone fracture until the fracture has healed but these pins are not used to secure a fixator structure to a bone.

Preferably the orthopaedic device comprises an adjusting means to axially adjust the securing means.

Preferably the threaded securing means of the inner shaft comprises a screw threaded section disposed substantially at one end of the inner shaft and being axially disposed substantially from the adjacent end of the outer tubular shaft.

Preferably the screw-threaded section of the inner shaft comprises substantially high profile threads due to a relatively thinner core diameter of the said inner shaft whereby, in use, the threads are suitable for withstanding predominately tensile (pull out) forces.

Preferably the outer tubular shaft has the threaded securing means in the form of a screw threaded section disposed substantially at one end of the outer tubular shaft.

Alternatively the outer tubular shaft has the threaded securing means in the form of a screw threaded section disposed at an intermediate position between the ends of the outer tubular shaft.

Preferably the screw threaded section of the outer tubular shaft comprises substantially low profile threads due to a relatively thicker core diameter of the said tubular shaft whereby, in use, the thicker core is capable of withstanding predominantly bending forces.

Preferably the inner shaft comprises an external threaded adjuster section disposed at the opposite end from said screw threaded section.

Preferably the adjusting means comprises a rotatable wheel having an internal thread which is engageable with the external threaded adjuster section of the inner shaft, whereby in use the wheel abuts the end of the outer tubular shaft, and causes the screw threaded sections to be urged axially relative to each other.

Preferably, the inner shaft comprises a disc like portion disposed at the opposite end to that of the securing means.

It is preferable that the fixator structure comprises at least one hole therethrough.

Preferably the inner shaft extends through the hole of the fixator structure where, in use, the fixator structure is clamped between the disc like portion of the inner shaft and the end of the outer tubular shaft.

A fixator structure is preferably of plate form.

The aspects of the invention may be carried into practice in various ways, and will now be described by way of example only with reference to the accompanying drawings in which:
Figure 1 shows a plan view of one form of a pin for the orthopaedic device;
Figure 2 shows a view through section A-A of the pin of Figure 1;
Figure 3 shows a sectional view of the pin of Figures 1 and 2 in use, secured to a bone structure and having an external bracing rod secured to one end of the orthopaedic device; and
Figure 4 is a sectional view, similar to that illustrated by Figures 1 to 3, which illustrates employing an internal bracing plate.

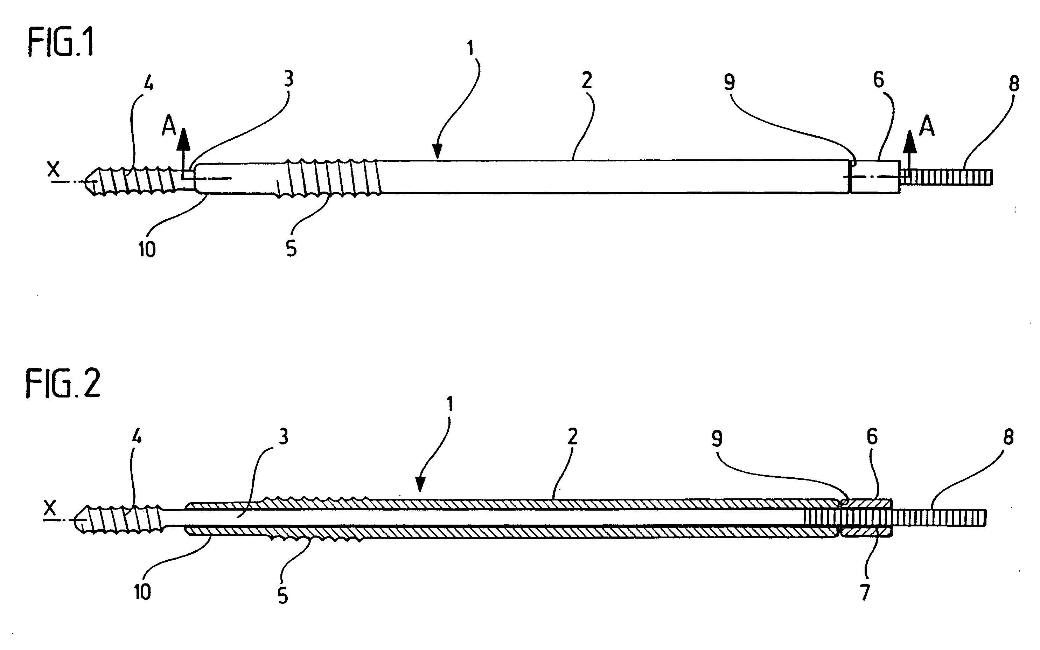

Referring to Figures 1 and 2, a pin 1 comprises a radially outer tubular shaft 2 and a radially inner shaft 3 extending coaxially therethrough, each shaft comprising a respective externally-threaded securing means comprising two screw threaded sections 4, 5 which are axially adjustable relative to each other. The outer tubular shaft 2 encases a substantial length of the inner shaft 3. The outer tubular shaft 2 and the inner shaft 3 are in slidable contact such that they may move relatively to each other whilst having the minimal radial clearance between the outermost longitudinal surface of the inner shaft 3 and the innermost longitudinal surface of the outer shaft 2.

The pin also comprises an adjusting means comprising a rotatable wheel 6 having an internal right-hand thread 7 being engageable with a corresponding external threaded adjuster section 8 of the inner shaft 3 disposed at the opposite end to said screw threaded section 4 whereby, in use, the wheel 6 abuts the end 9 of the outer tubular shaft 2. When the wheel is rotated about the common axis XX of the device and abuts the end 9, the relative axial distance between the two threaded sections 4, 5 is altered.

When the apparatus is assembled, as shown in Figures 1, 2 and 3, the screw threaded section 4 is disposed substantially at one end of the inner shaft 3 and is adjacent to the end 10 of the tubular shaft 2. The screw threaded section 4 has substantially high profile threads to help withstand axial (pull out) force.

The screw threaded section 5 is disposed from one end of the outer tubular shaft 2 and is adjacent to the end 10 of the tubular shaft 2. The end 10 extends from the section 5 towards the screw threaded section 4. The screw threaded section 5 has substantially thick core diameter to help withstand bending forces.

Conveniently, the diameter of the screw threaded section 4 is less than the diameter of the screw threaded section 5.

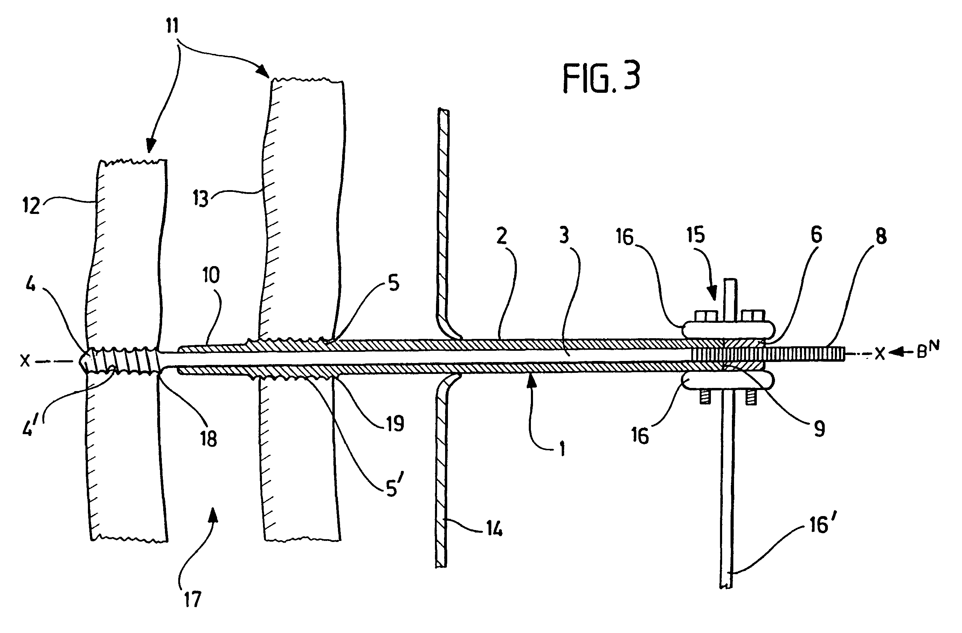

Referring to Figure 3 the pin 1, in use, is located within the bone structure 11 of a single bone.

The screw threaded section 4 is secured within the far cortex 12 of the bone structure 11 and the screw threaded section 5 is secured within the near cortex 13 of the bone structure 11. The bone structure 11 is of a tubular form having a substantially hollow chamber (medullary canal) 17 defined by the far cortex 12 and the near cortex 13.

The end 10 is disposed within the chamber 17 and substantially encases the section of the inner shaft 3 which is disposed axially between the screw threaded section 4 and the screw threaded section 5. The outer tubular shaft 2 extends axially from the near cortex 13 outwardly towards and through the skin 14 of the patient. The inner shaft 3 extends axially from the far cortex outwardly towards and through the skin 14 of the patient. The adjusting means is disposed from the outer surface of the skin 14.

As the wheel 6 is rotated about the common axis XX, by an operator, in a clockwise direction viewed from direction B, the inner shaft 3 experiences a tensile force and tends to move axially in an opposite direction to that of direction B. The rotation of wheel 6 also causes the outer tubular shaft 2 to experience a compressive force and tends to move axially in the direction B.

As the inner shaft 3 moves in the said opposite direction, it applies a force to the far cortex 12 via the contact between the screw threaded section 4 and the inner surface of the hole 4'. As the outer shaft 2 moves it applies a force to the near cortex 13 via the contact between the screw threaded section 5 and the inner surface of the hole 5'.

The threaded sections 4, 5 apply a compressive force to the inner surfaces of the respective holes 4', 5'.
This compressive force helps to prevent excessive movement of the threaded sections which leads to damage of the inner surface of the holes through the bone.

As the wheel 6 is rotated as described above, the device 1 applies a tangential compressive force through the bone structure 11 in the direction of the axis XX of the pin 1.

The said compressive force through the bone structure may be varied using the adjusting means. To increase the said compressive force the wheel 6 is further rotated clockwise as described above. To decrease the said compressive force the wheel 6 is rotated in the opposite direction (anti-clockwise) to that which is described above.

It will be appreciated that, if desired, the wheel can be rotated in an anti-clockwise direction to such an extent that the inner shaft 3 would experience a compressive force, the outer shaft 2 would experience a tensile force and hence the bone structure would experience a tangential tensile force in the direction of the axis XX of the pin 1.

The pin 1 can be secured to an existing or any other equivalent external fixator connecting rod system 15 comprising connecting rod system clamps 16 and connecting rod 16', in order for an external fixator configuration with four or more similar pins and a connecting rod to be mounted. Connecting rod system clamps 16 are attached and tightened to both the outer tubular shaft 2 and the rotatable wheel 6 at an appropriate distance from the skin of the patient 14, as seen in Figure 3.

The compressive force applied to the inner surfaces 4', 5' by the device 1 helps prevent the threaded sections 4, 5 becoming loose, with respect to the bone structure 11, and helps prevent the said sections from experiencing excessive movement. It will be appreciated that this provides more stability for the external fixator connecting rod system 15.

It will be appreciated that the pin 1 can also be used to hold together and compress sections of a fractured bone by securing the screw threaded section 4 within one section of the fractured bone and securing the screw threaded section 5 within the second section of the fractured bone. Compression can be applied on the two bone fragments using the adjusting means.

The procedure for inserting and assembling the pin 1 will now be described with reference to Figure 3.

First the hole 5' is made in the near cortex using standard orthopaedic drilling tools. At this stage the diameter of the hole 5' is slightly less than the diameter of the threaded section 5. Next the hole 4' is made in the far cortex. A smaller drill bit used to make the hole 4' passes through the hole 5' without touching the inner surface of the hole 5'. This means there is substantially less damage due to frictional heat between the drill and the surface of the hole 5'. Once the hole has been formed the drill bit is removed. To help ensure the inner surface of the hole 5' is not damaged by the drill a sleeve (not shown) may be fitted within the hole 5. The sleeve comprises a tubular section having a length substantially the same as the width of the near cortex and also having an outside diameter substantially equal to the diameter of the hole 5', at this stage, and also having an internal diameter substantially greater than the diameter of the drill so that the said drill will pass through the sleeve without substantially touching the inner surface of the said sleeve and the said sleeve will be held in position by the near cortex 13. At this stage the diameter of the hole 4' less than the diameter of the threaded section 4.

After the holes 4', 5' have been formed the inner shaft 3 is inserted into the patient and travels towards and through the hole 5' without substantially touching the inner surface of the hole 5'. The inner shaft 3 then passes through the hollow chamber 17 and the screw threaded section 4 engages the innermost opening 18 of the hole 4'. The inner shaft 4 is then rotated clockwise, with respect to direction B, and the threaded section 4 travels axially in the direction B through the hole 4'. As the threaded section 4 travels through the hole 4' the threads of the threaded section 4 are forced into the inner surface of the hole 4'.

Alternatively, the hole 4' is made using an adapted self-drilling screw threaded section 4. The adapted screw threaded section 4 comprises a sharpened drill type tip at the end thereof. Once the adapted screw threaded section 4 has travelled through the width of the far cortex 12 it is not removed and is left secured within the said cortex 12.

After the inner shaft 3 has been secured within the far cortex 12 the end 10 of the tubular shaft 2 is slid onto the outermost end of the external thread 8. The tubular shaft 2 is slid towards the hole 5'. The inner shaft 3 helps to guide the tubular shaft 2 towards the hole 5'. The diameter of the end 10 is less than the diameter of the hole 5' and passes through the said hole without engagement. The outer tubular shaft 2 travels towards the hole 5' until the screw threaded section 5 engages the outermost opening 19 of the hole 5'.

The outer tubular shaft 5 is then rotated clockwise, with respect to direction B, and the threaded section 5 travels axially in the direction B through the hole 5' until the tubular shaft 5 is sufficiently secure.

Once the shafts 4, 5 are secured to the far and near cortex respectively, the wheel 6 is threaded onto the external thread 8 until it abuts the outermost end 9 of the tubular shaft 2.

Once the desired number of pins has been inserted in the appropriate bone sites the ends of the pins protruding from the skin are connected to the external fixator connecting rod system 15.

As the wheel 6 is rotated in a clockwise direction, as described above, the device 1 applies a compressive force through the bone structure 11.

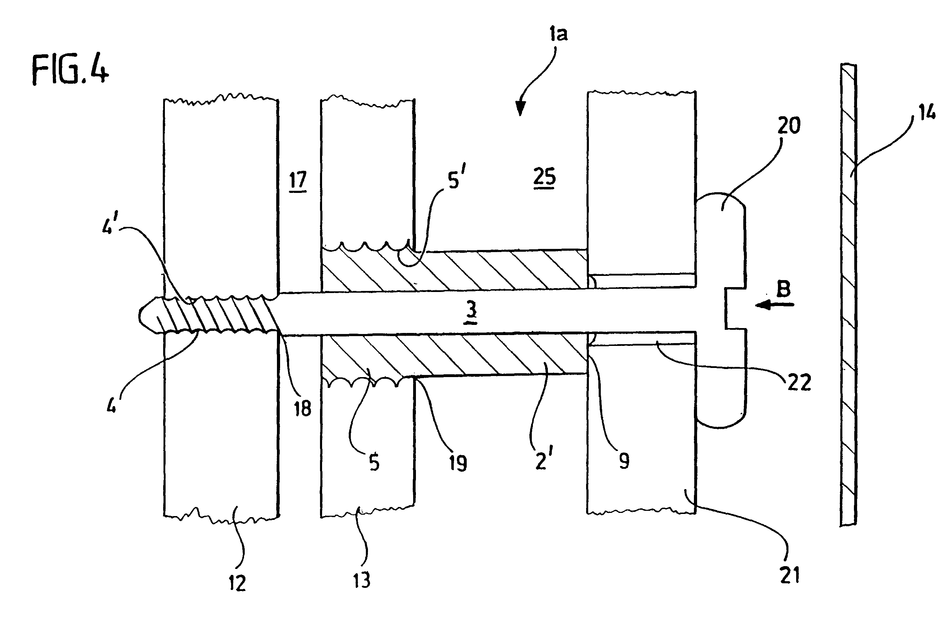

Figure 4 illustrates a modification which employs an internal fixator 21 in the form of a bracing plate.

Components and features corresponding to those shown in Figures 1 to 3 have been given corresponding reference numerals.

In Figure 4, inner shaft 3 is provided with head 20 whereby the shaft may be rotated, not only within the outer tubular shaft 2', but also within a hole 22 formed in the fixator plate 21. The inner surface of the head 20 bears against the outer surface of the plate 21.

The shaft 2' serves as a spacer between the near cortex 13 of the patient's bone and the plate 21, whereby the plate is secured at a predetermined distance 25 from the outer surface of the bone.

This is beneficial on two counts. Firstly, because the fixator plate 21 is kept clear of the bone, so that it does not introduce pressure necrosis problems. Secondly, the arrangement substantially reduces the need for cutting away muscle, with a view to bringing a fixator, such as a plate, into contact with the bone.

When the device 1a is in place, as shown in Figure 4, with the fixator plate 21 disposed between the head 20 and end 9 of the shaft 2', rotation of the head can take place so as to clamp the plate 21 securely, and thereby stabilise it in position.

In use, after holes 4', 5' have been formed, the outer tubular shaft 2' is slid towards the hole 5' until the screw threaded section 5 engages the outermost opening 19 of the hole 5'. The outer tubular shaft 2' is then rotated clockwise, with respect to direction B through the hole 5' until the section 5 is sufficiently secure.

After the outer tubular shaft 2' has been secured within the near cortex 13, the inner shaft 3 is inserted by it passing through the hole 22 of the orthopaedic plate 21 so as to travel towards and through the hole 5'. The inner shaft 3 is then passed through the hollow chamber 17 and the screw threaded section 4 made to engage the innermost opening 18 of the hole 4'. The inner shaft 4 is then rotated clockwise, with respect to direction B, and the threaded section 4 travels axially in the direction B through the hole 4'. As the threaded section 4 travels through the hole 4' the threads of the threaded section 4 grip the inner surface of the hole 4'.

For ease of assembly and use, the diameter of the threaded section 4 of the assembly of Figure 4 is made sufficiently small to pass through the bore of tubular shaft 2'.

The diameter of the outer end 9 of the tubular shaft 2' is made greater than the diameter of the hole 22 in fixator plate 21 to ensure that the plate 21 abuts end 9 on screwing-up of the head 20, and end 9 cannot pass through hole 22.

It will be appreciated that on screwing-up of the head 20 a compression force is generated between the ends 4, 5 to exert a compression force on the bore, and simultaneously a clamping force is applied to the plate 21 by the clamping force developed between head 20 and end 9.

## Claims

1. Orthopaedic device comprising one or more fixator structures and a plurality of pins adapted to be inserted into a bone of a patient so as to provide anchor points for one or more fixator structures (15, 21) adapted to be disposed within the patient's body and spaced from the patient's bones, **characterised in that** the pins employed comprise an outer tubular shaft (2; 2') and an inner shaft (3) extending coaxially therethrough, each shaft comprising a respective externally-threaded securing means (4, 5) for threadedly engaging with a bone, the securing means (4, 5) being axially adjustable relative to each other.

2. Orthopaedic device in accordance with claim 1, **characterised in that** it comprises an adjusting means (6, 7, 8) to axially adjust the securing means (4, 5).

3. Orthopaedic device in accordance with claim 1 or 2, **characterised in that** the threaded securing means (4) of the inner shaft (3) comprises a screw threaded section disposed substantially at one end of the inner shaft (3) and being axially disposed substantially from the adjacent end (10) of the outer tubular shaft (2, 2').

4. Orthopaedic device in accordance with claim 3, **characterised in that** the screw-threaded section (4) of the inner shaft (3) comprises substantially high profile threads due to a relatively thinner core diameter of the said inner shaft (3) whereby, in use, the threads are suitable for withstanding predominately tensile (pull out) forces.

5. Orthopaedic device in accordance with one of the preceding claims, **characterised in that** the outer tubular shaft (2, 2') has the threaded securing means (5) in the form of a screw threaded section disposed substantially at one end of the outer tubular shaft (2, 2').

6. Orthopaedic device in accordance with one of claims 1 to 4, **characterised in that** the outer tubular shaft (2) has the threaded securing means (5) in the form of a screw threaded section disposed at an intermediate position between the ends of the outer tubular shaft (2).

7. Orthopaedic device in accordance with one of claims 5 or 6, **characterised in that** the screw threaded section (5) of the outer tubular shaft (2, 2') comprises substantially low profile threads due to a relatively thicker core diameter of the said tubular shaft (2, 2') whereby, in use, the thicker core is capable of withstanding predominantly bending forces.

8. Orthopaedic device in accordance with one of claims 3 to 7, **characterised in that** the inner shaft (3) comprises an external threaded adjuster section (8) disposed at the opposite end from said screw threaded section (4).

9. Orthopaedic device in accordance with claim 8, **characterised in that** the adjusting means comprises a rotatable wheel (6) having an internal thread (7) which is engageable with the external threaded adjuster section (8) of the inner shaft (3), whereby in use the wheel (6) abuts the end of the outer tubular shaft (2), and causes the screw threaded sections (4, 5) to be urged axially relative to each other.

10. Orthopaedic device in accordance with one of the preceding claims, **characterised in that** the inner shaft (3) comprises a disc like portion (20) disposed at the opposite end to that of the securing means (4).

11. Orthopaedic device in accordance with one of the preceding claims, **characterised in that** the fixator structure (21) comprises at least one hole (22) therethrough.

12. Orthopaedic device in accordance with claim 11, **characterised in that** the inner shaft (3) extends through the hole (22) of the fixator structure (21) where, in use, the fixator structure is clamped between the disc like portion (20) of the inner shaft (3) and the end of the outer tubular shaft (2').

13. Orthopaedic device in accordance with one of the preceding claims, **characterised in that** the fixator structure (21) is of plate form.

14. Orthopaedic device in accordance with one of the claims 1 to 12, **characterised in that** the fixator structure (15) is a rod system.

## Patentansprüche

1. Orthopädische Vorrichtung umfassend eine oder mehrere Befestigungsstrukturen und eine Mehrzahl von Stiften, welche geeignet sind, um in einen Knochen eines Patienten eingesetzt zu werden, um so Verankerungspunkte für eine oder mehrere Befestigungsstrukturen (15, 21) vorzusehen, welche geeignet sind, um in dem Körper des Patienten angeordnet zu werden und welche von den Knochen des Patienten beabstandet sind, **dadurch gekennzeichnet, daß** die verwendeten Stifte einen äußeren rohrförmigen Schaft (2, 2') und einen inneren Schaft (3) umfassen, welcher sich koaxial dadurch erstreckt, daß jeder Schaft jeweils ein mit einem Außengewinde versehenes Sicherungsmittel (4, 5) umfaßt zum schraubenden Ineingriffbringen mit einem Knochen, daß die Sicherungsmittel (4, 5) axial relativ zueinander einstellbar sind.

2. Orthopädische Vorrichtung gemäß Anspruch 1, **dadurch gekennzeichnet, daß** sie ein Einstellmittel (6, 7, 8) umfaßt, um die Sicherungsmittel (4, 5) axial einzustellen.

3. Orthopädische Vorrichtung gemäß Anspruch 1 oder 2, **dadurch gekennzeichnet, daß** das mit einem Gewinde versehene Sicherungsmittel (4) des inneren Schafts (3) einen Schraubengewindeabschnitt umfaßt, welcher im wesentlichen an einem Ende des inneren Schafts (3) angeordnet ist und welcher axial im wesentlichen von dem benachbarten Ende (10) des äußeren rohrförmigen Schafts (2, 2') angeordnet ist.

4. Orthopädische Vorrichtung gemäß Anspruch 3, **dadurch gekennzeichnet, daß** der Schraubengewindeabschnitt (4) des inneren Schafts (3) im wesentlichen Hochprofil-Gewindegänge umfaßt aufgrund eines verhältnismäßig dünneren Kerndurchmessers des inneren Schafts (3), wonach, bei Verwendung, die Gewinde geeignet sind, um überwiegend Zugkräften (Herausziehen) standzuhalten.

5. Orthopädische Vorrichtung gemäß einem der voranstehenden Ansprüche, **dadurch gekennzeichnet, daß** der äußere rohrförmige Schaft (2, 2') die mit einem Gewinde versehenen Sicherungsmittel (5) in der Form eines Schraubengewindeabschnitts aufweist, welcher im wesentlichen an einem Ende des äußeren rohrförmigen Schafts (2, 2') angeordnet ist.

6. Orthopädische Vorrichtung gemäß einem der Ansprüche 1 bis 4, **dadurch gekennzeichnet, daß** der äußere rohrförmige Schaft (2) die mit einem Gewinde versehenen Sicherungsmittel (5) in der Form eines Schraubengewindeabschnitts aufweist, welcher in einer mittleren Position zwischen den Enden des äußeren rohrförmigen Schafts (2) angeordnet ist.

7. Orthopädische Vorrichtung gemäß einem der Ansprüche 5 oder 6, **dadurch gekennzeichnet, daß** der Schraubengewindeabschnitt (5) des äußeren rohrförmigen Schafts (2, 2') im wesentlichen Niedrigprofil-Gewindegänge umfaßt aufgrund eines verhältnismäßig dickeren Kerndurchmessers des rohrförmigen Schafts (2, 2'), wonach, bei Verwendung, der dickere Kern geeignet ist, um überwiegend Biegekräften standzuhalten.

8. Orthopädische Vorrichtung gemäß einem der Ansprüche 3 bis 7, **dadurch gekennzeichnet, daß** der innere Schaft (3) einen äußeren, mit einem Gewinde versehenen Einstellabschnitt (8) umfaßt, welcher an dem gegenüberliegenden Ende von dem Schraubengewindeabschnitt (4) angeordnet ist.

9. Orthopädische Vorrichtung gemäß Anspruch 8, **dadurch gekennzeichnet, daß** das Einstellmittel ein drehbares Rad (6) umfaßt, welches ein inneres Gewinde (7) aufweist, welches mit dem äußeren, mit einem Gewinde versehenen Einstellabschnitt (8) des inneren Schafts (3) in Eingriff bringbar ist, wonach, bei Verwendung, das Rad (6) an dem Ende des äußeren rohrförmigen Schafts (2) anliegt und bewirkt, daß die Schraubengewindeabschnitte (4, 5) axial relativ zueinander gedrängt werden.

10. Orthopädische Vorrichtung gemäß einem der voranstehenden Ansprüche, **dadurch gekennzeichnet, daß** der innere Schaft (3) einen scheibenartigen Bereich (20) umfaßt, welcher an dem gegenüberliegenden Ende zu dem des Sicherungsmittels (4) angeordnet ist.

11. Orthopädische Vorrichtung gemäß einem der voranstehenden Ansprüche, **dadurch gekennzeichnet, daß** die Befestigungsstruktur (21) mindestens ein Loch (22) dadurch umfaßt.

12. Orthopädische Vorrichtung gemäß Anspruch 11, **dadurch gekennzeichnet, daß** der innere Schaft (3) sich durch das Loch (22) der Befestigungsstruktur (21) erstreckt, wo, bei Verwendung, die Befestigungsstruktur zwischen dem scheibenartigen Bereich (20) des inneren Schafts (3) und dem Ende des äußeren rohrförmigen Schafts (2') geklemmt wird.

13. Orthopädische Vorrichtung gemäß einem der voranstehenden Ansprüche, **dadurch gekennzeichnet, daß** die Befestigungsstruktur (21) eine Plattenform aufweist.

14. Orthopädische Vorrichtung gemäß einem der Ansprüche 1 bis 12, **dadurch gekennzeichnet, daß** die Befestigungsstruktur (15) ein Stabsystem ist.

## Revendications

1. Dispositif orthopédique comprenant une ou plusieurs structures de fixateurs et une pluralité de broches conçues pour être insérées dans un os d'un patient de manière à fournir des points d'ancrage pour une ou plusieurs structures de fixateurs (15, 21) conçu pour être disposé dans le corps du patient et distant des os du patient, **caractérisé en ce que** les broches employées comprennent une tige tubulaire externe (2 ; 2') et une tige interne (3) qui s'étend coaxialement au travers de la précédente, chaque tige comprenant un moyen de fixation (4, 5) fileté extérieurement respectif pour coopérer par filetage avec un os, les moyens de fixation (4, 5) étant ajustables axialement l'un par rapport à l'autre.

2. Dispositif orthopédique selon la revendication 1, **caractérisé en ce qu'**il comprend un moyen d'ajustement (6, 7, 8) pour ajuster axialement les moyens de fixation (4, 5).

3. Dispositif orthopédique selon la revendication 1 ou 2, **caractérisé en ce que** le moyen de fixation fileté (4) de la tige interne (3) comprend une section à filetage de vis disposée sensiblement à une extrémité de la tige interne (3) et étant disposée axialement sensiblement depuis l'extrémité adjacente (10) de la tige tubulaire externe (2, 2').

4. Dispositif orthopédique selon la revendication 3, **caractérisé en ce que** la section à filetage de vis (4) de la tige interne (3) comprend des filets à profil sensiblement haut du fait d'un diamètre d'âme relativement plus petit de ladite tige interne (3), de sorte que, pendant l'utilisation, les filets sont appropriés pour résister à des forces principalement de tension (traction).

5. Dispositif orthopédique selon l'une des revendications précédentes, **caractérisé en ce que** la tige tubulaire externe (2, 2') comporte le moyen de fixation fileté (5) sous forme d'une section à filetage de vis disposée sensiblement à une extrémité de la tige tubulaire externe (2, 2').

6. Dispositif orthopédique selon l'une des revendications 1 à 4, **caractérisé en ce que** la tige tubulaire externe (2) comporte le moyen de fixation fileté (5) sous forme d'une section à filetage de vis disposée en une position intermédiaire entre les extrémités de la tige tubulaire externe (2).

7. Dispositif orthopédique selon l'une des revendications 5 et 6, **caractérisé en ce que** la section à filetage de vis (5) de la tige tubulaire externe (2, 2') comprend des filets de profil sensiblement bas du fait d'un diamètre d'âme relativement plus épaisse de ladite tige tubulaire (2, 2'), de sorte que, pendant l'utilisation, l'âme plus épaisse est capable de résister principalement à des forces de flexion.

8. Dispositif orthopédique selon l'une des revendications 3 à 7, **caractérisé en ce que** la tige interne (3) comprend une section d'ajustement (8) à filetage externe disposée à l'extrémité opposée par rapport à ladite section à filetage de vis (4).

9. Dispositif orthopédique selon la revendication 8, **caractérisé en ce que** le moyen d'ajustement comprend une couronne pouvant tourner (6) ayant un filet interne (7) pouvant coopérer avec la section d'ajustement à filetage externe (8) de la tige interne (3) de sorte que, pendant l'utilisation, la couronne (6) est en contact avec l'extrémité de la tige tubulaire externe (2) et amène les sections à filetage de vis (4, 5) à être poussées axialement l'une par rapport à l'autre.

10. Dispositif orthopédique selon l'une des revendications précédentes, **caractérisé en ce que** la tige interne (3) comprend une partie analogue à un disque (20) disposée à l'extrémité opposée à celle du moyen de fixation (4).

11. Dispositif orthopédique selon l'une des revendications précédentes, **caractérisé en ce que** la structure de fixateur (21) comprend au moins un trou (22) qui la traverse.

12. Dispositif orthopédique selon la revendication 11, **caractérisé en ce que** la tige interne (3) s'étend au travers du trou (22) de la structure de fixateur (21) de sorte que, pendant l'utilisation, la structure de fixateur est serrée entre la partie analogue à un disque (20) de la tige interne (3) et l'extrémité de la tige tubulaire externe (2').

13. Dispositif orthopédique selon l'une des revendications précédentes, **caractérisé en ce que** la structure de fixateur (21) est de forme plate.

14. Dispositif orthopédique selon l'une des revendications 1 à 12, **caractérisé en ce que** la structure de fixateur (15) est un système à barre.
